(19) Europäisches Patentamt
European Patent Office
Office européen des brevets

(11) EP 1 369 414 A1

(12) EUROPÄISCHE PATENTANMELDUNG

(43) Veröffentlichungstag:
**10.12.2003 Patentblatt 2003/50**

(21) Anmeldenummer: **03005233.6**

(22) Anmeldetag: **11.11.1997**

(51) Int Cl.[7]: **C07D 207/12**, C07D 211/40, C07D 409/12, A61K 31/40, A61K 31/4025, A61K 31/452, A61K 31/4535, A61P 11/00, A61P 21/00

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **11.11.1996 AT 197396**

(62) Dokumentnummer(n) der früheren Anmeldung(en) nach Art. 76 EPÜ:
**97911049.1 / 0 937 041**

(71) Anmelder:
- **Noe, Christian R.**
**1180 Wien (AT)**
- **Mutschler, Ernst**
**55129 Mainz (DE)**

(72) Erfinder:
- **Noe, Christian R.**
**1180 Wien (AT)**
- **Mutschler, Ernst**
**55129 Mainz (DE)**
- **Lambrecht, Günter**
**55129 Mainz (DE)**
- **Elgert, Michael**
**60437 Frankfurt/Main (DE)**
- **Czeche, Sittah**
**99867 Gotha (DE)**
- **Waelbroeck, Magali**
**1070 Brüssel (BE)**

(74) Vertreter: **Puchberger, Peter, Dipl.-Ing.**
**Puchberger, Berger & Partner**
**Reichsratsstrasse 13**
**1010 Wien (AT)**

Bemerkungen:
Diese Anmeldung ist am 10 - 03 - 2003 als Teilanmeldung zu der unter INID-Kode 62 erwähnten Anmeldung eingereicht worden.

(54) **Enantiomerenreine Arylcycloalkyl-Hydroxycarbonsäureester, Verfahren zu ihrer Herstellung und ihre Verwendung als Muskarinrezeptor-Modulatoren**

(57) Gegenstand der vorliegenden Erfindung sind enantiomerenreine Ester der allgemeinen Formel (I), worin $R_1$ einen mono- oder bicyclischen $C_5$-$C_7$-Cycloalkylrest, der gegebenenfalls durch einen oder mehrere $C_1$-$C_3$-Alkyl-, $C_2$-$C_6$-Alkenyl- oder $C_2$-$C_6$-Alkinylrest(e) und/oder durch ein oder mehrere Halogenatom(e) wie Fluor, Chlor, Brom oder lod substituiert ist; $R_2$ einen $C_1$-$C_6$-Alkyl-, $C_2$-$C_6$-Alkenyloder $C_2$-$C_6$-Alkinylrest, der gegebenenfalls durch ein oder mehrere Halogenatom (e) wie Fluor, Chlor, Brom, lod substituiert sein kann; $R_3$ einen $C_1$-$C_3$-Alkylrest, AR einen $C_6$-$C_{10}$-Aromaten oder einen Heteroaromaten, der Stickstoff, Schwefel oder Sauerstoff als Heteroatom enthält; n eine ganze Zahl 1 oder 2; $[A]^-$ ein Anion einer pharmakologisch unbedenklichen Säure bedeuten können, ausgenommen 3-[Cyclopentylhydroxyphenylacetyl)oxy]-1,1-dimethyl-pyrrolidinium (= Glycopyrronium), und in welchen OH, AR und R1, bei Blickrichtung vom quartären Kohlenstoff-Zentrum zur Carboxylgruppe hin, im Uhrzeigersinn angeordnet sind, sowie ihre Herstellung und ihre Verwendung in Arzneimitteln basierend auf ihrer interaktion mit muskarinischen Acetylchollnrezeptoren.

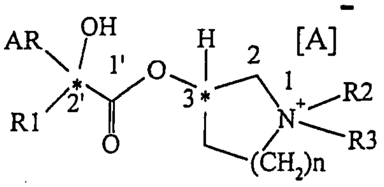

I

EP 1 369 414 A1

## Beschreibung

Gegenstand der vorliegenden Erfindung sind enantiomerenreine Ester der allgemeinen Formel I,

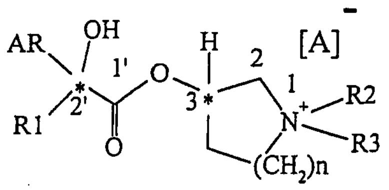

I

worin

$R_1$ einen mono- oder bicyclischen $C_5$-$C_7$-Cycloalkylrest, der gegebenenfalls durch einen oder mehrere $C_1$-$C_3$-Alkyl-, $C_2$-$C_6$-Alkenyl- oder $C_2$-$C_6$-Alkinylrest(e) und/oder durch ein oder mehrere Halogenatom(e) wie Fluor, Chlor, Brom oder lod substituiert ist;

$R_2$ einen $C_1$-$C_6$-Alkyl-, $C_2$-$C_6$-Alkenyl- oder $C_2$-$C_6$-Alkinylrest, der gegebenenfalls durch ein oder mehrere Halogenatom(e) wie Fluor, Chlor, Brom, lod substituiert sein kann;

$R_3$ einen $C_1$-$C_3$-Alkylrest,

AR einen $C_6$-$C_{10}$-Aromaten oder einen Heteroaromaten, der Stickstoff, Schwefel oder Sauerstoff als Heteroatom enthält;

n eine ganze Zahl 1 oder 2;

$[A]^-$ ein Anion einer pharmakologisch unbedenklichen Säure bedeuten können, ausgenommen 3-[Cyclopentylhydroxyphenylacetyl)oxy]-1,1-dimethyl-pyrrolidinium (= Glycopyrronium), und in welchen OH, AR und $R_1$, bei Blickrichtung vom quartären Kohlenstoff-Zentrum zur Carboxylgruppe hin, im Uhrzeigersinn angeordnet sind.

Besonders bevorzugt sind Verbindungen der allgemeinen Formel I, in der

$R_1$ einen Cyclopentyl- (wenn n = 2 oder AR = Thienyl), einen Cyclohexyl- oder einen Norbor nylrest;

$R_2$ einen Methylrest;

$R_3$ einen Methylrest;

AR einen Phenylrest oder Thienylrest;

n eine ganze Zahl 1 oder 2;

A Fluorid, Chlorid, Bromid oder lodid

bedeuten können und OH, AR und $R_1$ bei Blickrichtung gegen die Carboxylgruppe im Uhrzeigersinn angeordnet sind.

Die vorliegende Erfindung betrifft ferner Verfahren zur Herstellung der erfindungsgemäßen Verbindungen sowie ihre Verwendung als Arzneimittel.

$C_1$-$C_6$-Alkyl steht - sofern nicht anders definiert - für einen verzweigten oder unverzweigten Alkylrest mit 1 bis 6 Kohlenstoffatomen, der gegebenenfalls mit einem oder mehreren Halogenatomen substituiert sein kann. - Im Sinne der vorliegenden Erfindung sind beispielsweise folgende Substituenten als $C_1$-$C_6$-Alkylreste zu verstehen: Methyl, Ethyl, Propyl, 1-Methylethyl (*iso*-Propyl), Butyl, 1-Methylpropyl, 2-Methylpropyl, 1,1-Dimethylethyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Sofern nicht anders angegeben werden unter Alkylsubstituenten, die ein bis drei Kohlenstoffatome enthalten, vorzugsweise folgende Niederalkylreste verstanden:

Methyl, Ethyl, *n*-Propyl oder *iso*-Propyl.

$C_2$-$C_6$-Alkenyl steht - sofern nicht anders definiert - für einen verzweigten oder unverzweigten Alkylrest mit 2 bis 6 Kohlenstoffatomen, der eine oder gegebenenfalls zwei Doppelbindungen enthält gegebenenfalls mit einem oder mehreren Halogenatomen substituiert sein kann. - Im Sinne der vorliegenden Erfindung sind beispielsweise folgende Substituenten als $C_2$-$C_6$-Alkenylreste zu verstehen:

Vinyl, 2-Propenyl (Allyl), 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 3-Methyl-4-pentenyl, 4-Methyl-4-pentenyl, 1,1-Dimethyl-2-butenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, worunter der Allylrest bevorzugt ist.

$C_2$-$C_6$-Alkinyl steht - sofern nicht anders definiert - für einen verzweigten oder unverzweigten Alkylrest mit 2 bis 6 Kohlenstoffatomen, der eine oder gegebenenfalls zwei Dreifachbindungen oder eine Dreifachbindung und eine Doppelbindung enthalten kann und gegebenenfalls mit einem oder mehreren Halogenatomen substituiert sein kann. - Im Sinne der vorliegenden Erfindung sind beispielsweise folgende Substituenten als $C_2$-$C_6$-Alkinylreste zu verstehen:

2-Propinyl (Propargyl), 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 4-Methyl-2-pentinyl, 1-Methyl-3-pentinyl, 2-Methyl-3-pentinyl, 1-Methyl-4-pentinyl, 3-Methyl-4-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl oder 1-Ethyl-1-methyl-2-propinyl, worunter der Propargylrest bevorzugt ist.

Cycloalkyl steht im allgemeinen für einen gesättigten oder ungesättigten cyclischen Kohlenwasserstoffrest mit 3 bis 9 Kohlenstoffatomen, der gegebenenfalls mit einem Halogenatom oder mehreren Halogenatomen - vorzugsweise Fluor - substituiert sein kann, die untereinander gleich oder verschieden sein können. Bevorzugt sind cyclische Kohlenwasserstoffe mit 5 oder 6 Kohlenstoffatomen. Als Beispiele seien Cyclopropyl, Cyclobutyl, Cyclopentyl, Cyclopentenyl, Cyclohexyl, Cyclohexenyl, Cycloheptyl, Cycloheptenyl, Cycloheptadienyl, Cyclooctyl, Cyclooctenyl, Cyclooctadienyl und Cyclononinyl genannt.

Aryl steht im allgemeinen für einen aromatischen Rest mit 6 bis 10 Kohlenstoffatomen - auch in Zusammensetzungen, wobei der Aromat mit einer oder mehreren Niederalkylgruppe(n) mit 1 bis 3 Kohlenstoffatomen, Trifluormethylgruppe(n), Cyanogruppe(n), Alkoxygruppe(n), Nitrogruppe(n), Aminogruppe(n) und/oder einem oder mehreren Halogenatom(en) - untereinander gleich oder verschieden - substituiert sein kann; bevorzugter Arylrest ist ein gegebenenfalls substituierter Phenylrest, wobei als Substituenten Halogen - wie Fluor, Chlor oder Brom - Cyano sowie Hydroxyl bevorzugt sind.

Heteroaryl im Rahmen der oben angegebenen Definition steht im allgemeinen für einen 5- bis 6-gliedrigen Ring, der als Heteroatome Sauerstoff, Schwefel und/oder Stickstoff enthalten kann und an den ein weiterer aromatischer Ring ankondensiert sein kann. Bevorzugt sind 5- und 6-gliedrige aromatische Ringe, die einen Sauerstoff, einen Schwefel und/oder bis zu zwei Stickstoffatomen enthalten und die gegebenenfalls benzokondensiert sind.

Als besondere heterocyclische Systeme seien beispielsweise Acridinyl, Acridonyl, Alkylpyridinyl, Anthrachinonyl, Ascorbyl, Azaazulenyl, Azabenzanthracenyl, Azabenzanthrenyl, Azachrysenyl, Azacyclazinyl, Azaindolyl, Azanaphthacenyl, Azanaphthalenyl, Azaprenyl, Azatriphenylenyl, Azepinyl, Azinoindolyl, Azinopyrrolyl, Benzacridinyl, Benzazapinyl, Benzofuryl, Benzonaphthyridinyl, Benzopyranonyl, Benzopyranyl, Benzopyronyl, Benzochinolinyl, Benzochinolizinyl, Benzothiepinyl, Benzothiophenyl, Benzylisoquinolinyl, Bipyridinyl, Butyrolactonyl, Caprolactamyl, Carbazolyl, Carbolinyl, Catechinyl, Chromenopyronyl, Chromonopyranyl, Cumarinyl, Cumaronyl, Decahydrochinolinyl, Decahydrochinolonyl, Diazaanthracenyl, Diazaphenanthrenyl, Dibenzazapinyl, Dibenzofuranyl, Dibenzothiphenyl, Dichromylenyl, Dihydrofuranyl, Dihydroisocumarinyl, Dihydroisochinolinyl, Dihydropyranyl, Dihydropyridinyl, Dihydropyridonyl, Dihydropyronyl, Dihydrothiopyranyl, Diprylenyl, Dioxanthylenyl, Oenantholactamyl, Flavanyl, Flavonyl, Fluoranyl, Fluoresceinyl, Furandionyl, Furanochromanyl, Furanonyl, Furanochinolinyl, Furanyl, Furopyranyl, Furopyronyl, Heteroazulenyl, Hexahydropyrazinoisoquinolinyl, Hydrofuranyl, Hydrofuranonyl, Hydroindolyl, Hydropyranyl, Hydropyridinyl, Hydropyrrolyl, Hydrochinolinyl, Hydrothiochromenyl, Hydrothiophenyl, Indolizidinyl, Indolizinyl, Indolonyl, Isatinyl, Isatogenyl, Isobenzofurandionyl, Isobenzfuranyl, Isochromanyl, Isoflavonyl, Isoindolinyl, Isoindolobenzazapinyl, Isoindolyl, Isochinolinyl, Isochinuclidinyl, Lactamyl, Lactonyl, Maleimidyl, Monoazabenzonaphthenyl, Naphthalenyl, Naphthimidazopyridindionyl, Naphthindolizinedionyl, Naphthodihydropyranyl, Naphthofuranyl, Naphthyridinyl, Oxepinyl, Oxindolyl, Oxolenyl, Perhydroazolopyridinyl, Perhydroindolyl, Phenanthrachinonyl, Phthalideisoquinolinyl, Phthalimidyl, Phthalonyl, Piperidinyl, Piperidonyl, Prolinyl, Parazinyl, Pyranoazinyl, Pyranoazolyl, Pyranopyrandionyl, Pyranopyridinyl, Pyranochinolinyl, Pyranopyrazinyl, Pyranyl, Pyrazolopyridinyl, Pyridinethionyl, Pyridinonaphthalenyl, Pyridinopyridinyl, Pyridinyl, Pyridocolinyl, Pyridoindolyl, Pyridopyridinyl, Pyridopyrimidinyl, Pyridopyrrolyl, Pyridochinolinyl, Pyronyl, Pyrrocolinyl, Pyrrolidinyl, Pyrrolizidinyl, Pyrrolizinyl, Pyrrolodioazinyl, Pyrrolonyl, Pyrrolopyrmidinyl, Pyrrolochinolonyl, Pyrrolyl, Chinacridonyl, Chinolinyl, Chinolizidinyl, Chinolizinyl, Chinolonyl, Chinuclidinyl, Rhodaminyl, Spirocumaranyl, Succinimidyl, Sulpholanyl, Sulpholenyl, Tetrahydrofuranyl, Tetrahydroisochinolinyl, Tetrahydropyranyl, Tetrahydropyridinyl, Tetrahydrothiapyranyl, Tetrahydrothiophenyl, Tetrahydrothipyranonyl, Tetrahydrothipyranyl, Tetronyl, Thiaphenyl, Thiachromanyl, Thiadecalinyl, Thianaphthenyl, Thiapyranyl, Thiapyronyl, Thiazolopyridinyl, Thienopyridinyl, Thienopyrrolyl, Thienothiophenyl, Thiepinyl, Thiochromenyl, Thiocumarinyl, Thiopyranyl, Triazaanthracenyl, Triazinoindolyl, Triazolopyridinyl, Tropanyl, Xanthenyl, Xanthonyl, Xanthydrolyl, Adeninyl, Alloxanyl, Alloxazinyl, Anthranilyl, Azabenzanthrenyl, Azabenzonaphthenyl, Azanaphthacenyl, Azaphenoxazinyl, Azapurinyl, Azinyl, Azoloazinyl, Azolyl, Barbituric Acid, Benzazinyl, Benzimidazolethionyl, Benzimidazolonyl, Benzisothiazolyl, Benzisoxazolyl, Benzocinnolinyl, Benzodiazocinyl, Benzodioxolanyl; Benzodioxolyl, Benzopyridazinyl, Benzothiazepinyl, Benzothiazinyl, Benzothiazolyl, Benzoxazinyl, Benzoxazolinonyl, Benzoxazolyl, Cinnolinyl, Depsidinyl, Diazaphenanthrenyl,

Diazepinyl, Diazinyl, Dibenzoxazepinyl, Dihydrobenzimidazolyl, Dihydrobenzothiazinyl, Dihydrooxazolyl, Dihydropyridazinyl, Dihydropyrimidinyl, Dihydrothiazinyl, Dioxanyl, Dioxenyl, Dioxepinyl, Dioxinonyl, Dioxolanyl, Dioxolonyl, Dioxopiperazinyl, Dipyrimidopyrazinyl, Dithiolanyl, Dithiolenyl, Dithiolyl, Flavinyl, Furopyrimidinyl, Glycocyamidinyl, Guaninyl, Hexahydropyrazinoisoquinolinyl, Hexahydropyridazinyl, Hydantoinyl, Hydroimidazolyl, Hydroparazinyl, Hydropyrazolyl, Hydropyridazinyl, Hydropyrimidinyl, Imidazolinyl, Imidazolyl, Imidazoquinazolinyl, Imidazothiazolyl, Indazolebenzopyrazolyl, Indoxazenyl, Inosinyl, Isoalloxazinyl, Isothiazolyl, Isoxazolidinyl, Isoxazolinonyl, Isoxazolinyl, Isoxazolonyl. Isoxazolyl, Lumazinyl, Methylthyminyl, Methyluracilyl, Morpholinyl, Naphthimidazolyl, Oroticyl, Oxathianyl, Oxathiolanyl, Oxazinonyl, Oxazolidinonyl, Oxazolidinyl, Oxazolidonyl, Oxazolinonyl, Oxazolinyl, Oxazolonyl, Oxazolopyrimidinyl, Oxazolyl, Perhydrocinnolinyl, Perhydropyrroloazinyl, Perhydropyrrolothiazinyl, Perhydrothiazinonyl, Perimidinyl, Phenazinyl, Phenothiazinyl, Phenoxathiinyl, Phenoxazinyl, Phenoxazonyl, Phthalazinyl, Piperazindionyl, Piperazinodionyl, Polyquinoxalinyl, Pteridinyl, Pterinyl, Purinyl, Pyrazinyl, Pyrazolidinyl, Pyrazolidonyl, Pyrazolinonyl, Parazolinyl, Pyrazolobenzodiazepinyl, Pyrazolonyl, Pyrazolopyrimidinyl, Pyrazolotriazinyl, Pyrazolyl, Pyridazinyl, Pyridazonyl, Pyridopyrazinyl, Pyridopyrimidinyl, Pyrimidinethionyl, Pyrimidinyl, Pyrimidionyl, Pyrimidoazepinyl, Pyrimidopteridinyl, Pyrrolobenzodiazepinyl, Pyrrolodiazinyl, Pyrrolopyrimidinyl, Chinazolidinyl, Chinazolinonyl, Chinazolinyl, Chinoxalinyl, Sultamyl, Sultinyl, Sultonyl, Tetrahydrooxazolyl, Tetrahydropyrazinyl, Tetrahydropyridazinyl, Tetrahydroquinoxalinyl, Tetrahydrothiazolyl, Thiazepinyl, Thiazinyl, Thiazolidinonyl, Thiazolidinyl, Thiazolinonyl, Thiazolinyl, Thiazolobenzimidazolyl, Thiazolyl, Thienopyrimidinyl, Thiazolidinonyl, Thyminyl, Triazolopyrimidinyl, Uracilyl, Xanthinyl, Xylitolyl, Azabenzonapththenyl, Benzofuroxanyl, Benzothiadiazinyl, Benzotriazepinonyl, Benzotriazolyl, Benzoxadiazinyl, Dioxadiazinyl, Dithiadazolyl, Dithiazolyl, Furazanyl, Furoxanyl, Hydrotriazolyl, Hydroxytrizinyl, Oxadiazinyl, Oxadiazolyl, Oxathiazinonyl, Oxatriazolyl, Pentazinyl, Pentazolyl, Petrazinyl, Polyoxadiazolyl, Sydonyl, Tetraoxanyl, Tetrazepinyl, Tetrazinyl, Tetrazolyl, Thiadiazinyl, Thiadiazolinyl, Thiadiazolyl, Thiadioxazinyl, Thiatriazinyl, Thiatriazolyl, Thiatriazolyl, Triazepinyl, Triazinoindolyl, Triazinyl, Triazolinedionyl, Triazolinyl, Triazolyl, Trioxanyl, Triphenodioxazinyl, Triphenodithiazinyl, Trithiadiazepinyl, Trithianyl, oder Trioxolanyl genannt.

Als pharmazeutisch geeignetes Salz wird das Salz einer pharmakologisch unbedenklichen Säure bezeichnet.

Weiterer Gegenstand der Erfindung ist ein Verfahren zur Herstellung dieser Verbindungen, bei welchem eine vorzugsweise tertiäre Aminoalkoholkomponente in enantiomerenreiner Form und die Säure in enantiomerenreiner oder bevorzugt razemischer Form zum Ester umgesetzt wird und abschließend die Quarternierung unter Verwendung eines geeigneten Alkylierungsmittels, vorzugsweise eines Alkylhalogenids, erfolgt. In der bevorzugten Verfahrensvariante erfolgt nach Einbringung der razemischen Säure in den Ester die Trennung der entstehenden Diastereomere durch Kristallisation. Weiterer Gegenstand der Erfindung ist die Anwendung der enantiomerenreinen Ester der allgemeinen Formel I in Arzneimitteln.

Die erfindungsgemäßen enantiomerenreinen Verbindungen der allgemeinen Formel I können nach Verfahren hergestellt werden, die an sich aus dem Stand der Technik bekannt sind. Die essentiellen Schritte des Herstellungsverfahrens bestehen insbesondere darin, daß man eine enantiomerenreine α-Hydroxycarbonsäure der allgemeinen Formel II in welcher OH, AR und R1, bei Blickrichtung vom quartären Kohlenstoff-Zentrum zur Carboxylgruppe hin, im Uhrzeigersinn angeordnet sind und

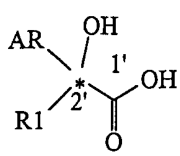

II

worin $R_1$ und AR die zuvor genannte Bedeutung haben, oder ihren Ester, bevorzugt einen $C_1$ - $C_3$ Alkylester, oder ein aktiviertes Säurederivat mit einem enantiomerenreinen Aminoalkohol der allgemeinen Formel III (*R*- oder *S*-Enantiomer)

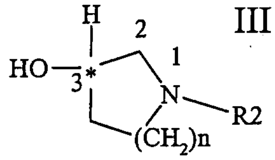

III

worin $R_2$ und n die zuvor genannte Bedeutung haben, umsetzt und die so erhaltenen enantiomerenreinen Ester der allgemeinen Formel IV

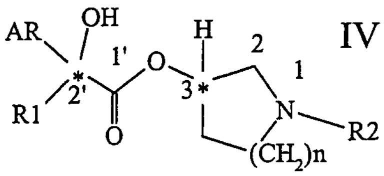

IV

mit einem Alkylierungsmittel der allgemeinen Formel

R3 - X,

in der X eine gegen eine tertiäre Aminogruppe substituierbare Austrittsgruppe verkörpert, umsetzt und das resultierende Salz isoliert bzw. umsalzt.

Das bevorzugte Verfahren ist dadurch gekennzeichnet, daß man eine racemische α-Hydroxycarbonsäure der allgemeinen Formel II

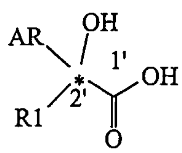

II

worin R1 und AR die zuvor genannte Bedeutung haben, oder ihren Ester oder ein aktiviertes Säurederivat mit einem enantiomerenreinen Aminoalkohol der allgemeinen Formel III (*R*- oder *S*-Enantiomer)

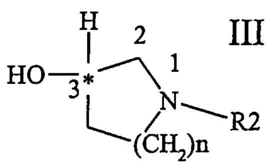

III

worin R2 und n die zuvor genannte Bedeutung haben, umsetzt, das resultierende Diastereomerengemisch nach an sich aus dem Stand der Technik bekannten Verfahren - insbesondere auf dem Wege der Kristallisation, bevorzugt unter Verwendung einer enantiomerenreinen Hilfssäure - trennt, die so erhaltenen Ester der allgemeinen Formel IV, in welchen OH, AR und R1, bei Blickrichtung vom quartären Kohlenstoff-Zentrum zur Carboxylgruppe hin, im Uhrzeigersinn angeordnet sind

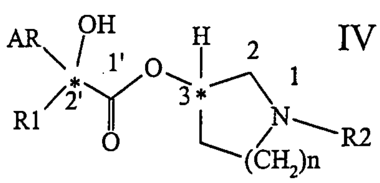

IV

mit einem Alkylierungsmittel der allgemeinen Formel

R3 - X,

in der X eine gegen eine tertiäre Aminogruppe substituierbare Austrittsgruppe verkörpert, umsetzt und das resultierende Salz isoliert bzw. umsalzt.

Ester von Aryl-cycloalkyl-hydroxysäuren mit cyclischen Alkoholen, in welchen ein quartärer Stickstoff vorhanden ist, und welche mit der allgemeinen Formel I

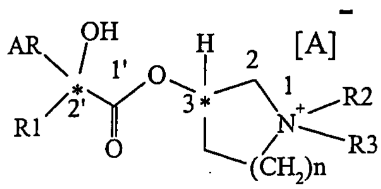

I

beschrieben werden, bestehend aus einer Hydroxycarbonsäure, in welcher AR einen aromatischen Ring bedeutet und in welcher $R_1$ einen cycloaliphatischen Ring bedeutet und bestehend aus einer Alkoholkomponente, in welcher sich die Hydroxylgruppe an einem Dimethylpyrrolidiniumring (n=1) oder Dimethylpiperidiniumring (n=2) befindet, in welchem $R_2$=$R_3$ ein Niederalkyl bedeutet, und in welcher A ein Halogenid bedeutet, wurden in einigen Fällen als Spasmolytika beschrieben. Wenn die beiden Reste $R_2$ und $R_3$ identisch sind, weisen Verbindungen der allgemeinen Formel I zwei Chiralitätszentren auf. Das eine Zentrum ist dem Säureteil zuzuordnen und betrifft die mit 2' bezeichnete Position, das zweite Chiralitätszentrum befindet sich im cyclischen Ringsystem an der mit 3 bezeichneten Position. Da Verbindungen dieser Struktur somit zwei Chiralitätszentren aufweisen, sind im Prinzip vier Stereoisomere (3R,2'R; 3S,2'R; 3R,2'S und 3S,2S') denkbar. Bisher wurden reine Stereoisomere der allgemeinen Formel I weder isoliert noch synthetisch hergestellt, oder-was für den Gegenstand der vorliegenden Erfindung grundlegend ist - pharmakologisch untersucht. Der wichtigste, auch in der Therapie eingesetzte Vertreter der allgemeinen Formel I ist Glycopyrroniumbromid (AR = Phenyl, $R_1$ = Cyclopentyl, $R_2$=$R_3$ = Methyl, n = 1, A = Br). Mit dem internationalen Freinamen Glycopyrroniumbromid wird das razemische Diastereoisomerengemisch, in welchem alle vier Stereoisomere enthalten sind, beschrieben.

Bisher bekanntgewordene Publikationen und Patente befassen sich entweder mit dem als Stereoisomerengemisch vorliegenden Wirkstoff Glycopyrroniumbromid (CAS 596-51-0), mit erythro-(RN 59677-73-5) bzw. threo- (RN 59677-70-2) konfigurierten Racematen des tert. Aminoesters (CRN 131118-11-1), welche lediglich als Vorstufe bei der Synthese der Verbindungen der Formel I angesehen werden können, oder dem Stereoisomerengemisch des analogen Cyclohexyl-Derivates ($R_1$ = Cyclohexyl) der allgemeinen Formel I (mit n = 1) (RN 101564-29-8). In den Publikationen zu den Chemical Abstracts Zitaten 80:53209h und 80:53214f sind Ergebnisse von Kristallstrukturanalysen der als Stereoisomerengemische vorliegenden Verbindungen Glycopyrroniumbromid und Hexapyrroniumbromid beschrieben. Die Publikationen zu den Chemical Abstracts Zitaten 80:66587e, 80:66588f und 89:191258 beschreiben Ergebnisse von pharmakologischen Untersuchungen mit dem Stereoisomerengemisch der Verbindung Glycopyrroniumbromid bzw. von Kombinationspräparaten dieser Substanz mit Neostigmin und Pyridostygmin. Die Publikationen zu den Abstracts 84:43164h und 85:32832u beschreiben die teilweise Trennung des Stereoisomerengemisches auf dem Wege einer Kristallisation mit 5-Nitroisophthalsäure und die NMR-Untersuchung der bereits oben erwähnten threo- bzw. erythro-konfigurierten Racemate. Hierbei gelang den Autoren, ausgehend vom Stereoisomerengemisch (CRN 131118-11-1), lediglich die Diastereomerentrennung in die zwei Razemate, jedoch keine Herstellung der enantiomerenreinen Verbindungen. Die Publikationen zu den Chemical Abstracts Zitaten 96:29498m, 105:48928x, 113:158782t, 89:191258k, sowie das europäische Patent EP 128886 A2 beschreiben Ergebnisse von Studien zur chromatographischen Analytik des Stereoisomerengemisches der Verbindung Glycopyrroniumbromid bzw. die Herstellung der verwendeten stationären Phasen. In keiner der aufgeführten Publikationen wird eine Enantiomerentrennung bzw. Isolierung der einzelnen Stereoisomere der allgemeinen Formel 1 berichtet. Eine HPL-chromatographische Trennung gelang in allen aufgeführten Fällen nur auf der Stufe der Diastereomeren. Die Herstellung der erfindungsgemäß beanspruchten enantiomerenreinen Verbindungen der allgemeinen Formel I ist aus dem Stand der Technik noch nicht bekannt.

Die pharmakologische Wirkung von Arzneistoffen der allgemeinen Formel I basiert auf ihrer Interaktion mit muskarinischen Acetylcholinrezeptoren (Muskarinrezeptoren). Sie werden daher als m-Cho-linozeptor-Antagonisten, bzw. Parasympatholytika oder - wegen ihrer erschlaffenden Wirkung auf die glatte Muskulatur - als neurotrope Spasmolytika bezeichnet. Die vielfältigen Wirkungen der Parasympatholytika umfassen: Beschleunigung der Herzfrequenz, Reduktion der Sekretion der Tränen-, Speichel- und Schweiß-Sekretion sowie der Drüsen des Verdauungstraktes, Erschlaffung der glatten Muskulatur der Bronchien, des Magen-Darm-Kanals, der Gallenwege, Uteren und der Harnblase, Erweiterung der Pupillen und Akkomodationsstörung. Quartäre Spasmolytika, zu welchen auch die Verbindungen gemäß der allgemeinen Formel I gehören, überwinden die Blut-Hirn-Schranke nicht und sind daher zentral unwirksam. Je nach Art der Anwendung sind die gewünschten und unerwünschten Wirkungen von Parasympatholytika verschieden. Verwendet man diese Substanzen als Spasmolytika, so wird man beispielsweise die verminderte Speichelsekretion oder die Pupillenerweiterung als Nebenwirkung bezeichnen.

Aufgrund der Forschungen der letzten Jahre ist es bekannt, daß Muskarinrezeptoren keine einheitliche Struktur besitzen, sondern daß die pharmakologischen Wirkungen auf Interaktionen mit mindestens vier verschiedenen Muskarinrezeptor-Subtypen zurückzuführen sind. Diese weisen einerseits eine unterschiedliche Verteilung in verschiedenen Organen auf, andererseits sind bei manchen neuronalen Signalübertragungskaskaden verschiedene Muskarinrezeptor-Subtypen mit verschiedenen Funktionen involviert. Verschiedene Wirkungen bzw. Nebenwirkungen lassen sich auf Interaktionen mit den verschiedenen Rezeptorsubtypen zurückführen, so daß eine hohe Subtypspezifität ein Ziel bei der Entwicklung moderner Spasmolytika ist. Glycopyrroniumbromid ist ein lange etablierter Wirkstoff, der den Anforderungen eines" modernen" Therapeutikums dieses Typs nicht entspricht. Glycopyrroniumbromid ist jedoch nicht nur ein Razemat, sondern darüber hinaus ein Diastereomerengemisch, bei welchem je nach Herstellungsprozedur die Verhältnisse der einzelnen Isomere im Produkt sogar schwanken können. Somit kann es bei solchen isomeren Wirkstoffgemischen zu zufälligen Subtypprofilen kommen, wodurch ein gezielter Einsatz erschwert und das Auftreten von unerwünschten Nebenwirkungen provoziert wird.

Häufig liegt bei der Trennung des Razemates eines Arzneistoffes in Enantiomere die pharmakologische Wirkung ausschließlich bei einem der Enantiomere. Bei Verbindungen gemäß der vorliegenden Erfindung können alle Isomere im Prinzip Rezeptoraffinität aufweisen. Jedoch zeigen die einzelnen Enantiomere zum einen deutliche Unterschiede in ihren Affinitäten, zum anderen ergeben sich auch deutliche Abweichungen in der Subtypspezifität $M_1$ - $M_4$, wobei die Unterschiede in den Affinitäten maximal einen Faktor von etwa 1000 ausmachen. Gerade die hohe Affinität zum $M_3$-Rezeptor-Subtyp bei einer relativ niederen Affinität zum $M_2$-Rezeptor-Subtyp macht die beanspruchten höher affinen Enantiomere, bei denen im Säureteil OH, AR und R1, bei Blickrichtung zur Carboxylgruppe, im Uhrzeigersinn angeordnet sind, zu besonders geeigneten Wirkstoffen zur Therapie von Spasmen der glatten Muskulatur des Magen-Darm-Traktes und des Urogenitaltraktes sowie zur Behandlung obstruktiver Atemwegserkrankungen. Durch ein besonders günstiges Subtypprofil und eine durch ihre hohe Affinität bedingte besonders niedrige Dosierungsmöglichkeit bringen sie einen effizienteren therapeutischen Erfolg bei deutlich reduziertem Nebenwirkungspotential.

Ein weiterer besonders wichtiger Faktor bei der therapeutischen Anwendung von Enantiomeren der allgemeinen Formel I besteht in der kinetischen Subtypselektivität. Beispielsweise liegen die Dissoziationshalbwertzeiten von (3S,2'S)-, (3S,2'R)-, (3R,2'R)- und (3R,2'S)-3-[(Cyclopentylhydroxyphenylacetyl)oxy]-1,1-dimethyl-pyrrolidinium-iodid (d.h. Verbindungen der allgemeinen Formel I mit AR = Phenyl, $R_1$ = Cyclopentyl, $R_2$ = $R_3$ = Methyl, n = 1, A = I) beim $M_3$-Rezeptorsubtyp zwischen 1 Minute und 120 Minuten, während die Dissoziationshalbwertszeiten an den Subtypen $M_1$, $M_2$ und $M_4$ im Bereich von wenigen Minuten liegen. Gerade Verbindungen mit einer besonders langen Dissoziationshalbwertszeit erlauben wegen ihrer starken Haftung eine besonders niedere Dosierung bei lange anhaltendem therapeutischem Effekt. Die Möglichkeit, durch gezielte Auswahl eines Enantiomers mit bestimmter Dissoziationshalbwertszeit die Dauer der pharmakologischen Wirkung gezielt zu beeinflussen, stellt einen weiteren wichtigen Fortschritt der Verbindungen der vorliegenden Erfindung gegenüber dem Stand der Technik dar. Die beschriebenen Eigenschaften waren nicht vorhersehbar, auch gab es keine Hinweise in der Literatur.

Zusammenfassend läßt sich feststellen, daß sich enantiomerenreine Ester der allgemeinen Formel I gegenüber dem Stand der Technik durch ihre pharmakodynamische Selektivität auszeichnen. Sie besitzen in der bevorzugt beanspruchten Konfiguration eine deutlich höhere Affinität zu muskarinischen $M_3$- als zu $M_2$-Rezeptoren und zeigen darüber hinaus eine kinetische Selektivität für $M_3$-Rezeptoren, d.h. sie diffundieren nur langsam von diesem Rezeptortyp. Aufgrund dieser Eigenschaften eignen sie sich ganz besonders zur Therapie von Spasmen der glatten Muskulatur des Magen-Darm-Kanals und des Urogenitaltraktes sowie zur Behandlung obstruktiver Atemwegserkrankungen, wie Asthma bronchiale und chronische Bronchitis. Im Vergleich zu den bisher angewandten, nichtselektiven Parasympatholytika weisen sie aufgrund ihrer definierten Subtypselektivität deutliche Unterschiede in den pharmakologischen Eigenschaften auf. Im Vergleich zu bekannten Stereoisomerengemischen oder Razematen können die Verbindungen zudem in besonders niedriger Dosierung eingesetzt werden (Vermeidung von enantiomerem Ballast!). Aus diesen Gründen sind Nebenwirkungen mit Sicherheit in deutlich geringerem Umfang zu erwarten.

Demgemäß ist die Verwendung von enantiomerenreinen Estern der allgemeinen Formel I in Arzneimitteln zur Therapie von Spasmen der glatten Muskulatur des Magen-Darm-Kanals und des Urogenitaltraktes sowie zur Behandlung obstruktiver Atemwegserkrankungen (Asthma bronchiale, chronische Bronchitis) ein bevorzugter Gegenstand der Erfindung. Besonders bevorzugt ist die Verwendung von Enantiomeren der allgemeinen Formel I mit hoher $M_3$-Subtypselektivität ($pK_i$ größer als 10) und großen Dissoziationshalbwertszeiten am $M_3$-Rezeptor in Arzneimitteln zur Behandlung obstruktiver Atemwegserkrankungen, bevorzugt Asthma bronchiale und chronische Bronchitis.

Die beschriebene Erfindung wird nunmehr durch die folgenden Beispiele erläutert. Verschiedenartige, andere Ausgestaltungen werden für den Fachmann aus der vorliegenden Beschreibung ersichtlich. Es wird jedoch ausdrücklich darauf hingewiesen, daß die Beispiele und die Beschreibung lediglich zur Erläuterung vorgesehen und nicht als Einschränkung der Erfindung anzusehen sind.

**Beispiele:**

**Beispiel 1 (Referenzbeispiel):**

Herstellung von (3S,2'S) 3-[(cyclopentylhydroxyphenylacetyl)oxy]-1,1-dimethyl-pyrrolidinium-iodid 1a (allg. Formel I, Ar = Phenyl, $R_1$ = Cyclopentyl, $R_2$ = $R_3$ = Methyl, n=1, A = I)

In einer trockenen Reaktionsapparatur werden 20 mmol (3S)-1-Methyl-3-pyrrolidinol und 24 mmol 2-Cyclopentyl-2-hydroxy-phenylessigsäuremethylester in 500 ml n-Heptan abs. vorgelegt. Anschließend werden 200 ml Heptan zum Entfernen aller Feuchtigkeitsspuren überdestilliert und durch den Wasserabscheider abgelassen. Nach dem Abkühlen werden 2 mmol NaH oder NaOMe (10 mol%) zugesetzt und wiederum zum Sieden erhitzt. Die Temperatur wird so gewählt, daß das n-Heptan nur langsam überdestilliert. Die übergehende Menge wird über 5-6 h fortlaufend aus dem Tropftrichter ersetzt, bis der Hydroxyester vollständig umgesetzt ist. Nach wäßriger Aufarbeitung des Reaktionsgemisches und Extraktion mit Ether wird das Rohprodukt über $Na_2SO_4$ / $K_2CO_3$ 2:1 getrocknet. Nach Absaugen vom Trockenmittel wird im Eisbad vorgekühlt und unter Eiskühlung bis zur Sättigung mit etherischer HCl / 2-Butanon versetzt. Hierbei fällt das Produkt zunächst ölig an. Durch Zusatz von 2-Butanon bzw. durch Abdestillieren von Ether erhält man eine klare Lösung, aus der unter Eiskühlung das Hydrochlorid des Diasteromerengemisches (3S,2'R/S)-3-(2-cyclopentylhydroxy-phenylacetoxy)-1-methyl-pyrrolidin (allg. Formel IV Ar = Phenyl, $R_1$ = Cyclopentyl, $R_2$= Methyl, n = 1) auskristallisiert. Ausbeute 15,7 mmol. Fp. 176°C.

Herstellung der Hydrogentartrate: Diastereomerentrennung durch fraktionierende Kristallisation:

Zur Herstellung der Hydrogentartrate überführt man 15 mmol des oben beschriebenen Hydrochlorids mit $NaHCO_3$ / $K_2CO_3$ Puffer pH 10 in einen Scheidetrichter und extrahiert die wäßrige Phase dreimal mit je 150 ml Diethylether. Die vereinten etherischen Phasen werden mit 100 ml Ethylacetat versetzt und über $Na_2SO_4$ / $K_2CO_3$ 2:1 getrocknet . Nach Absaugen vom Trockenmittel wird am Rotationsverdampfer auf ca. 100 ml Volumen reduziert. Die Lösung wird auf ca. 60°C erhitzt und mit einer Lösung von 1,2 eq (18mmol) enantiomerenreiner Weinsäure in Ethylacetat versetzt. Das Hydrogentartrat kristallisiert über Nacht im Kühlschrank. Durch mehrfaches Umkristallisieren lassen sich die Diastereomeren bis zu einem de von 99% trennen. Ausbeute 8,3 mmol.
(3S,2'S) 3-[(cyclopentylhydroxyphenylacetyl)oxy]-1-methyl-pyrrolidin, D-(-)-Hydrogentartrat allg. Formel IV (AR = Phenyl, $R_1$ = Cyclopentyl, $R_2$ = Methyl, n = 1): Smp.178-179°C $^1$H-NMR-Spektrum (300 MHz $CD_4O$): δ (ppm) 1,3-1,7 (M, 8H,Cyclopentyl-$CH_2$), 2,05-2,1 (M,1H,C4), 2,39-2,46 (M,1H,C4), 2,77 (S,3H,N-Methyl), 2,97-3,0 (M,1H,Cyclopentyl C1), 3,18-3,25 (dd,1H,C2, $^2$J =12,8 Hz,$^3$J=0-1 Hz), 3,31-3,5 (M,2H,C5), 3,6-3,7 (dd,1H,3J=5,2Hz,2J=13Hz,C2), 4,42 (S,2H,Tartrat), 5,34-5,39 (M,1H,C3), 7,2-7,8 (M,5H) Zuordnung aufgrund H,H-COSY-NMR

Quarternierung:

Nach Freisetzen der Basen durch Extraktion mit Ether gegen Bicarbonat-Puffer pH 10 und Trocknen über $Na_2SO_4$ / $K_2CO_3$ 2:1 wird durch Zugabe von 3 eq (20mmol) Methyliodid quarterniert und das kristallin anfallende Produkt (3S,2'S)-3-[(cyclopentylhydroxyphenylacetyl)oxy]-1,1-dimethyl-pyrrolidinium-iodid 1a (allg. Formel I, AR = Phenyl, $R_1$ = Cyclopentyl, $R_2$ = $R_3$ = Methyl, n = 1, A = I) abgesaugt. (Die Bestimmung der Diastereomerenüberschüsse kann durch HPLC-Methoden an β-Cyclodextrin- und "Whelck"-Phasen bzw. durch Auswertung von NMR-Spektren der oben beschriebenen Hydrogentartrate erfolgen.)

(3S,2'S)-3-[(cyclopentylhydroxyphenylacetyl)oxy]-1,1-dimethyl-pyrrolidinium-iodid (1a) allg. Formel I (AR = Phenyl, $R_1$ = Cyclopentyl, $R_2$ = $R_3$ = Methyl, n = 1, A = I) Smp.165°C $^1$H-NMR (300MHz $CD_4O$) δ (ppm) (1,15-1,4 (M, 2H), 1,5-1,7 (M,6H),Cyclopentyl-Methylen), 2,2 (M,1H,C 4), 2,63 (M,1H,C 4), 2,9 (S,3H,N-Methyl), 2,93-2,99 (M,1H, Cyclopentyl-Methin), 3,1 (S,3H,N-Methyl), 3,43-3,47 (dd,1H,C2,$^2$J=14Hz,$^3$J=0Hz), 3,5-3,7 (M,2H,C5), 3,75 (dd,1H, C2,$^2$J=13,7Hz,$^3$J=6,05Hz), 5,38 (M,1H,C3), 7,15-7,4 (M,3H), 7,5-7,65 (M,2H) $^{13}$C-NMR 52 MHz $CD_4O$ δ (ppm) (24,4-25,4) (4t,Cyclopentyl-methylen), 28,5 (t,C4), 47,4 (t,Cyclopentyl-Methin), 51,3 (q,N-Methyl), 51,8 (q,N-Methyl), 63,6 (t,C5), 68,9 (t,C2), 72,0 (d,C3), 78,4 (s,Hydroxyester C2), 124,5 (d), 126,1 (d), 126,7 (d), 140,5 (s), 172,4 (s)

Die Bestimmung der Diastereomerenreinheit (de) erfolgte durch Vergleich der Integrale der N-Methyl-protonen der diastereomeren Hydrogentartrate.in $^1$H-NMR-Spektren (300 MHz, $CD_4O$), bzw. durch HPLC-Analytik an β-Cyclodextrinphasen. (Cyclobond β-CD-OH, 50*0,4 cm, Puffer: 85%$H_2O$, 15% CH3CN, 0,2% CH3COOH V/V, 0,35 ml/ min isokratisch, UV-Detektion: 236 nm).

**Beispiel 2 (Referenzbeispiel):**

Herstellung von (3S,2'S) 3-[(cyclohexylhydroxyphenylacetyl)oxy]-1,1-dimethyl-pyrrolidiniumbromid 2a (allg. Formel I, Ar = Phenyl, $R_1$ = Cyclohexyl, $R_2$ = $R_3$ = Methyl, n=1, A = Br)

Die Herstellung der unter Beispiel 2 aufgeführten Verbindung erfolgt ausgehend von (3S)-1-Methyl-3-pyrrolidinol, 2-Cyclohexyl-2-hydroxy-phenylessigsäuremethylester und NaOMe nach dem unter Beispiel 1 beschriebenen Verfahren. Umesterung und Diastereomerentrennung der 3S-konfigurierten L (+)-Hydrogentartrate erfolgen in analoger Art und Weise.

(3S,2'S)- 3-[(cyclohexylhydroxyphenylacetyl)oxy]-1-methyl-pyrrolidin, L(+)-hydro-gentartrat: allg. Formel IV (AR = Phenyl, $R_1$ = Cyclopentyl, $R_2$ = Methyl, n = 1): $^1$H-NMR (300 MHz CD4O): δ (ppm) 1,12-1,2 (M,4H,Cyclohexyl-$CH_2$), 1,2-1,64 (M,3H,Cyclohexyl-$CH_2$), 1,64-1,67 (M,2H,Cyclohexyl-$CH_2$), 1,75-1,85 (M,1H,Cyclohexyl-$CH_2$), 2,03-2,1 (M,1H,C4), 2,25-2,4 (M,1H,Cyclohexyl-Methin), 2,40-2,55 (M,1H,C4), 2,78 (S,3H,N-Methyl), 3,22 (dd,1H, C2, $^2$J =13,3 Hz,$^3$J=0-1 Hz), 3,27-3,51 (M,2H,C5), 3,65 (dd,1H,3J=5,42Hz, 2J=13, 2Hz,C2), 4,42 (S,2H,Tartrat), 5,37 (M,1H,C3), 7,2-7,61 (M,5H). Die Zuordnung erfolgte aufgrund H,H-COSY-NMR-Spektren. $^{13}$C-NMR (50 MHz $CD_4O$ / DEPT): δ (ppm) (26,71 (t), 27,35(t), 27,47 (t), 28,71 (t),Cyclohexyl-Methylen), 31,8 (t,C4), 42,5 (q,N-Methyl), 46,92 (d, Cyclohexyl-methin), 55,35 (t,C5), 61,24 (t,C2), 74,2 (d,Tartrat-Methin), 75,2 (d,C3), 82,7 (s,Hydroxyester C2'), 126,9 (d), 128,7 (d), 129,2 (d), 142,1 (s), 175,0 (s,Tartrat-Carboxyl), 177,2 (s,Hydroxyester-Carboxyl).

Zur Quarternierung wird in Abwandlung der unter Beispiel 1 aufgeführten Vorschrift Methylbromid in tert.Butylmethylether verwendet.

(3S, 2'S) 3-[(cyclohexylhydroxyphenylacetyl)oxy]-1,1-dimethyl-pyrrolidiniumbromid 2a: $^1$H-NMR (300 MHz CD4O): δ (ppm) (1,1-2,0 (M,10H), Cyclohexyl-Methylen), 2,1-2,3 (M,1H,cHex-Methin), 2,35-2,45 (M,1H,C4), 2,65-2,85 (M,1H,C4), 3,07 (S,3H,N-Methyl), 3,21 (S,3H,N-Methyl), 3,55-3,8 (M,3H,C2,C5), 3,85 (dd,1H, C2,$^2$J=13,8Hz,$^3$J=6,1Hz), 5,47 (M,1H,C3), 7,25-7,4 (M,3H), 7,55-7,65 (M,2H)

Die Bestimmung der Diastereomerenreinheit (de) erfolgte durch Vergleich der Integrale der N-Methyl-protonen der diastereomeren Hydrogentartrate.bei 2,78 ppm und 2,82 ppm in $^1$H-NMR-Spektren (300 MHz, $CD_4O$).

**Beispiel 3:**

Herstellung von (3S,2'R) 3-[(cyclohexylhydroxyphenylacetyl)oxy]-1,1-dimethyl-pyrrolidiniumbromid 2b (allg.Formel I, Ar = Phenyl, $R_1$ = Cyclohexyl, $R_2$ = $R_3$ = Methyl, n = 1, A = Br)

Aus den Mutterlaugen der Diastereomerentrennung zur Herstellung von (3S,2'S) 3-[(cyclohexylhydroxyphenylacetyl)oxy]-1,1-dimethyl-pyrrolidiniumbromid (Beispiel 2) wird nach Freisetzen des tertiären Aminoesters die diastereomere Verbindung (3S,2'R)- 3-[(cyclohexylhydroxyphenylacetyl)oxy]-1-methyl-pyrrolidin, D(-)-Hydrogentartrat kristallisiert.

(3S,2'R) 3-[(cyclohexylhydroxyphenylacetyl)oxy]-1-methyl-pyrrolidin, D(-)-Hydrogen-tartrat: $^1$H-NMR (300 MHz CD4O): δ (ppm) 1,12-1,2 (M,4H,Cyclohexyl-$CH_2$), 1,2-1,64 (M,3H,Cyclohexyl-$CH_2$), 1,64-1,67 (M,2H,Cyclohexyl-$CH_2$), 1,75-1,85 (M,1H,Cyclohexyl-$CH_2$), 2,03-2,08 (M,1H,C4), 2,25-2,4 (M,1H,Cyclohexyl-Methin), 2,40-2,55 (M, 1H,C4), 2,82 (S,3H,N-Methyl), 3,27-3,34 (M,2H,C5,C2), 3,44 (M,1H,C5), 3,73 (dd,1H,3J=5,45Hz,2J=13,3Hz,C2), 4,42 (S,2H,Tartrat), 5,37 (M,1H,C3), 7,2-7,61 (M,5H) Zuordnung aufgrund H,H-COSY-NMR. $^{13}$C-NMR (62,5 MHz $CD_4O$): δ (ppm) (25,31 (t), 26(t), 26,1 (t), 27,32 (t),Cyclohexyl-Methylen), 30,57 (t,C4), 41,1 (q,N-Methyl), 45,6 (d,Cyclohexyl-Methin), 53,93 (t,C5), 59,7 (t,C2), 72,8 (d,Tartrat-Methin), 73,82 (d,C3), 81,3 (s,Hydroxyester C2'), 125,54 (d), 127,2 (d), 127,8 (d), 140,85 (s), 173,64 (s,Tartrat-Carboxyl), 175,8 (s,Hydroxyester-Carboxyl)

Nach Quarternierung mit Methylbromid in tert.Butyl-Methylether erhält man (3S,2'R) 3-[(cyclohexylhydroxyphenylacetyl)oxy]-1,1-dimethyl-pyrrolidiniumbromid. $^1$H-NMR (300MHz $CD_4O$): δ (ppm) (1,1-1,8 (M,10H), Cyclohexyl-Methylen), 2,2-2,4 (M,2H,C4&cHex-Methin), 2,65-2,85 (M,1H,C 4), 3,03 (S,3H,N-Methyl), 3,21 (S,3H,N-Methyl), 3,55-3,8 (M, 3H, C2, C5), 3,86 (dd,1H,C2,$^2$J=13,8Hz,$^3$J=6,1Hz), 5,48 (M,1H,C3), 7,25-7,4 (M,3H), 7,57-7,65 (M,2H). Die Bestimmung der Diastereomerenreinheit (de) erfolgte wie in Beispiel 2 beschrieben.

**Beispiel 4:**

Herstellung von (3R,2'R) 3-[(cyclohexylhydroxyphenylacetyl)oxy]-1,1-dimethyl-pyrrolidiniumbromid 2c (allg. Formel I, Ar = Phenyl, $R_1$ = Cyclohexyl, $R_2$ = $R_3$ = Methyl, n = 1, A = Br)

Herstellung der unter Beispiel 4 aufgeführten Verbindung erfolgt ausgehend von (3R)-1-Methyl-3-pyrrolidinol, 2-Cyclohexyl-2-hydroxy-phenylessigsäuremethylester und NaOMe nach dem unter Beispiel 1 beschriebenen Verfahren. Umesterung und Diastereomerentrennung der 3R-konfigurierten L-(+)-Hydrogentartrate erfolgen in analoger Art

und Weise. Zur Quarternierung wird analog der unter Beispiel 3 aufgeführten Vorschrift Methylbromid in tert.Butyl-Methylether verwendet. [$^{1}$H]- und [$^{13}$C]-NMR Analytik identisch zu der in Beispiel 2 aufgeführten (3S,2'S)-konfigurierten Verbindung.
Die Bestimmung der Diastereomerenreinheit (de) erfolgte wie in Beispiel 2 beschrieben.

2a 2b 2c 2d

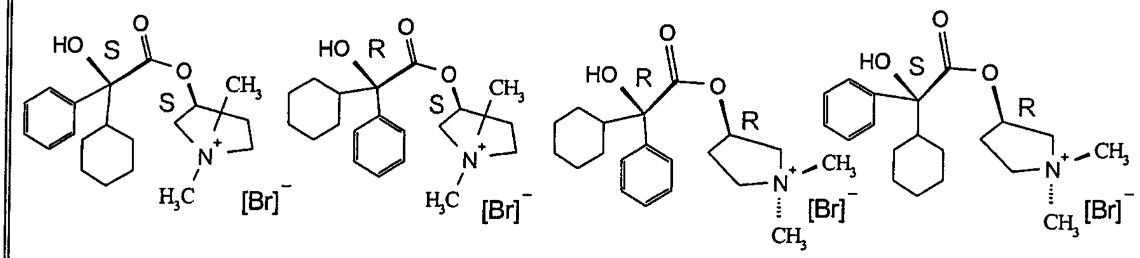

Formeln der Enantiomere 2a - 2d

**Beispiel 5:**

Herstellung von (3*R*,2'*S*)-3-[(2'-Cyclopentyl-2'-hydroxy-2'-(2''-thienyl)acetyl)oxy]-1,1-dimethylpyrrolidiniumbromid (allg. Formel I, Ar = 2-Thienyl, R1 = Cyclopentyl, R2 = R3 = Methyl, n = 1, $A^-$ = $Br^-$)

In einer trockenen Reaktionsapparatur werden 5 mmol (3R)-1-Methyl-3-pyrrolidinol und 5 mmol (2R)-2-Cyclopentyl-2-hydroxy-2-(2'-thienyl)essigsäuremethylester in 150 ml n-Heptan abs. vorgelegt. Anschließend werden 100 ml Heptan zum Entfernen aller Feuchtigkeitsspuren destillativ entfernt und durch den Wasserabscheider abgelassen. Nach dem Abkühlen werden 0,5 mmol NaOMe (10 mol%) zugesetzt und wiederum zum Sieden erhitzt. Die übergehende Menge Lösungsmittel wird über 5-6 h fortlaufend mittels Tropftrichter ersetzt. Nach wässriger Aufarbeitung des Reaktionsgemisches und Extraktion mit Ether wird die organische Phase über $Na_2SO_4$ / $K_2CO_3$ 2:1 getrocknet. Entfernen von Trocken- und Lösungsmittel liefert die freie Base.

Quarternierung:

Die freie Base wird durch Zugabe von 3 eq Methylbromid, gelöst in tert-Butylmethylether, quarterniert und das kristallin anfallende Produkt abgesaugt.
$^{1}$H-NMR (300 MHz, $D_2O$): δ = 7.33 (d, 1H, Thienyl); 7.13 (d, 1H, Thienyl); 6.96 (dd, 1H, Thienyl); 5.50-5.42 (m, 1H); 4.67 (s, $H_2O$); 3.78-3.62 (m, 2H); 3.58-3.47 (m, 2H); 3.08 (s, 3H, N-$CH_3$, (3 *R*,2'*S*)); 3.01-2.83 (m, 4H, N-$CH_3$, (3*R*,2'*S*) und Cyclopentyl-Methin); 2.76-2.62 (m, 1H); 2.37-2.22 (m, 1H (3*R*,2'*S*)); 1.65-1.20 (m, 8H, Cyclopentyl-Methylen).
Die dazu diastereomeren Verbindungen unterscheiden sich im wesentlichen durch die Verschiebungen folgender Signale: 3.11 (s, $NCH_3$); 3.04 (s, $NCH_3$); 2.15-2.03 (m). $^{13}$C-NMR (50 MHz, $D_2O$): δ = 176.2 (s, 1'-COO); 147.7 (s, Thienyl); 130.1 (d, Thienyl,; 128.8 (d, Thienyl); 128.4 (d, Thienyl); 82.2 (s); 76.9 (d); 72.9 (t); 67.7 (t); 56.4 (q, $NCH_3$); 55.8 (q, $NCH_3$); 49.4 (d, Cyclopentyl-Methin); 32.5 (t); 29.2/29.0/28.7/28.4 (t, Cyclopentyl-Methylen)
Summenformel / Masse des Kations: $(C_{17}H_{26}NO_3S)^+$ $(Br)^-$ / 324.47
(ESI+)-Massenspektrum : 324.4 = $M^+$.
Die Bestimmung der Absolutkonfiguration erfolgte mittels Röntgenstrukturanalyse.

**Beispiel 6:**

(3*R*,2'*S*)-3-[(2'-Cyclopentyl-2'-hydroxy-2'-(2''-thienyl)acetyl)oxy]-1,1-dimethylpiperidiniumbromid (allg. Formel I, Ar = 2-Thienyl, R1 = Cyclopentyl, R2 = R3 = Methyl, n = 2, $A^-$ = $Br^-$)
Die Herstellung erfolgt nach der unter Beispiel 5 angegebenen allgemeinen Vorschrift aus (3*R*)-Methylpiperidinol und (2*S*)-2-Cyclopentyl-2-hydroxy-2-(2'-thienyl)essigsäuremethylester $^{1}$H-NMR (300 MHz, $CDCl_3$): δ = 7.27 ($CHCl_3$); 7.24 (dd, 1H, Thienyl); 7.12 (dd, 1H, Thienyl); 6.97 (dd, 1H, Thienyl); 5.26-5.20 (m, 1H); 4.40 (dd, 1H); 4.25-4.12 (m, 1H); 3.86-3.76 (m, 1H); 3.61-3.52 (m, 1H); 3.51 (s, 3H, N-$CH_3$); 3.0 (s, 3H, N-$CH_3$); 2.88-2.74 (m, 1H, Cyclopentyl-Methin);

2.38-2.23 (m, 1H); 2.11-1.90 (m, 3H); 1.76-1.40 (m, 8H, Cyclopentyl-Methylen).
$^{13}$C-NMR (50 MHz, $CDCl_3$): δ = 173.3 (s, 1'-COO); 145.6 (s, Thienyl); 127.2 (d, Thienyl); 125.4 (d, Thienyl); 125.0 (d, Thienyl); 78.6 (s); 68.4 (d); 62.7 (t); 61.5 (t); 56.3 (q, $NCH_3$); 51.4 (q, $NCH_3$); 47.5 (d, Cyclopentyl-Methin); ) 26.9/26.7/26.4/26.0 (t, Cyclopentyl-Methylen); 25.0 (t); 16.7 (t) Summenformel / Masse des Kations: $(C_{18}H_{28}NO_3S)^+$ $(Br)^-$ / 338.49
(ESI+)-Massenspektrum: 337.9 = $M^+$.
Elementaranalyse: Ber.: C 51,67 H 6,75 N 3,35; Gef.: C 51,44 H 6,92 N 3,10

**Beispiel 7:**

(3 *R*,2' *R*)-3-[(2'-Cyclopentyl-2'-hydroxy-2'-phenylacetyl)oxy]-1,1-dimethylpiperidiniumbromid (allg. Formel I, Ar = Phenyl, R1 = Cyclopentyl, R2 = R3 = Methyl, n = 2, $A^-$ = $Br^-$) Die Herstellung erfolgt nach der unter Beispiel 5 angegebenen allgemeinen Vorschrift aus (3R)-Methylpiperidinol und (2 *R*)-2-Cyclopentyl-2-hydroxy-2-phenylessigsäuremethylester $^1$H-NMR (300 MHz, $CDCl_3$): δ = 7.57 (d, 2H, Phenyl); 7.36 (dd, 2H, Phenyl); 7.30-7.23 (m, darin 1H, Phenyl und $CHCl_3$ [7.27]); 5.22-5.16 (m, 1H); 4.32 (dd, 1H); 4.18-4.05 (m, 1H); 3.75-3.64 (m, 1H); 3.58-3.47 (m, 1H); 3.43 (s, 3H, $NCH_3$, (3 *R*,2' *R*)); 3.0-2.85 (m, 1H, Cyclopentyl-Methin); 2.80 (s, 3H, $NCH_3$, (3 *R*,2' *R*)); 2.31-2.18 (m, 1H); 1.99-1.82 (m, 3H); 1.75-1.32 (m, 8H, Cyclopentyl-Methylen) Die dazu diastereomeren Verbindungen unterscheiden sich im wesentlichen durch die Verschiebungen folgender Signale: 3.49 (s, $NCH_3$); 3.27 (s, $NCH_3$).
$^{13}$C-NMR (50 MHz, $CDCl_3$): δ = 174.0 (s, 1'-COO); 140.9 (s, Phenyl); 128.4 (d, Phenyl); 127.8 (d, Phenyl); 125.9 (d, Phenyl); 79.4 (s); 67.7 (d); 62.6 (t); 61.4 (t); 56.1 (q, $NCH_3$); 51.3 (q, $NCH_3$); 45.8 (d, Cyclopentyl-Methin); 26.9/26.5/26.3/25.9 (t, Cyclopentyl-Methylen); 24.8 (t); 16.6 (t) Summenformel / Masse des Kations: $(C_{20}H_{30}NO_3)^+$ $(Br)^-$ / 332.47
(ESI+)-Massenspektrum: 331.9 = $M^+$
Elementaranalyse: Ber.: C 58,25 H 7,33 N 3,40; Gef.: C 58,03 H 7,21 N 3,33

**Beispiel 8:**

(3*R*,2'*R*)-3-[(2'-Cyclopentyl-2'-hydroxy-2'-(p-fluorphenyl)acetyl)oxy]-1,1-dimethylpyrrolidiniumbromid (allg. Formel I, Ar = para-Fluorphenyl, R1 = Cyclopentyl, R2 = R3 = Methyl, n = 1, $A^-$ = $Br^-$)
Die Herstellung erfolgt nach der unter Beispiel 5 angegebenen allgemeinen Vorschrift aus (3*R*)-Methylpyrrolidinol und (2*R*)- 2-Cyclopentyl-2-hydroxy-2-(4'-fluorphenyl)essigsäuremethylester $^1$H-NMR (300 MHz, $D_2O$): δ = 7.53 (dd, 2H, Phenyl); 7.06 (dd, 2H, Phenyl); 5.47-5.35 (m, 1H); 4.68 (s, $H_2O$); 3.73-3.59 (m, 2H); 3.57-3.39 (m, 2H); 3.10-2.97 (m, 4H, darin: Cyclopentyl-Methin und N-$CH_3$ [bei 3.06]); 2.81 (s, 3H, N-$CH_3$); 2.76-2.62 (m, 1H); 2.33-2.20 (m, 1H); 1.65-1.32 (m, 7H, Cyclopentyl-Methylen); 1.21-1.06 (m, 1H).
$^{13}$C-NMR (50 MHz, $D_2O$): δ = 176.9 (s, 1'-COO); 164.9 (d, Phenyl); 139.2 (d, Phenyl); 130.9 (dd, Phenyl); 118.0 (dd, Phenyl); 82.8 (s); 76.7 (d); 72.9 (t); 67.7 (t); 56.3 (q, $NCH_3$); 55.7 (q, $NCH_3$); 47.8 (d, Cyclopentyl-Methin); 32.5 (t, 4-C); 29.2/28.8/28.7/28.4 (t, Cyclopentyl-Methylen) Summenformel / Masse des Kations: $(C_{19}H_{27}NO_3F)^+$ $(Br)^-$ / 336.43
(ESI+)-Massenspektrum: 336.3 = $M^+$
Elementaranalyse: Ber.: C 54,64 H 6,54 N 3,36; Gef.: C 54,64 H 6,49 N 3,27

**Beispiel 9:**

(3*R*,2'*R*)-3-[(2'-Cyclopentyl-2'-hydroxy-2'-(p-fluorphenyl)acetyl)oxy]-1,1-dimethylpiperidiniumbromid (allg. Formel I, Ar = para-Fluorphenyl, R1 = Cyclopentyl, R2 = R3 = Methyl, n = 2, $A^-$ = $Br^-$)
Die Herstellung erfolgt nach der unter Beispiel 5 angegebenen allgemeinen Vorschrift aus (3R)-Methylpiperidinol und (2R)-2-Cyclopentyl-2-hydroxy-2-(4'-fluorphenyl)essigsäuremethylester

$^1$H-NMR (300 MHz, $CDCl_3$): δ = 7.58 (dd, 2H, Phenyl); 7.27 ($CHCl_3$); 7.07 (dd, 2H, Phenyl); 5.25-5.16 (m, 1H); 4.28 (dd, 1H); 4.12-3.99 (m, 1H); 3.82-3.67 (m, 2H); 3.48 (s, 3H, N-$CH_3$); 3.10 (s, 3H, N-$CH_3$); 2.92-2.79 (m, 1H, Cyclopentyl-Methin); 2.28-2.12 (m, 1H); 2.00-1.85 (m, 3H); 1.74-1.31 (m, 8H, Cyclopentyl-Methylen).
$^{13}$C-NMR (50 MHz, $CDCl_3$): δ = 173.9 (s, 1'-COO); 162.11 (d, Phenyl); 136.8 (d, Phenyl); 127.8 (dd, Phenyl); 115.3 (dd, Phenyl); 79.2 (s); 77.2 (d); 62.7 (t); 61.6 (t); 55.8 (q, $NCH_3$); 51.8 (q, $NCH_3$); 46.5 (d, Cyclopentyl-Methin); 27.0/26.5/26.3/25.9 (t, Cyclopentyl-Methylen); 25.0 (t); 16.8 (t) Summenformel / Masse des Kations: $(C_{20}H_{29}NO_3)^+$ $(Br)^-$ / 350.46
(ESI+)-Massenspektrum: 350.1 = $M^+$,
Elementaranalyse: Ber.: C 55,82 H 6,79 N 3,25; Gef.: C 55,94 H 6,71 N 3,10

**Beispiel 10:**

(3*R*,2'*R*)- 3-[(Cyclopentylhydroxyphenylacetyl)oxy]-1-allyl-1-methyl-pyrrolidinium-bromid allg.Formel I (AR = Phenyl, $R_1$ = Cyclopentyl, $R_2$ = Allyl, $R_3$ = Methyl, n = 1, A = Br);
Die Herstellung erfolgt nach der unter Beispiel 5 angegebenen allgemeinen Vorschrift aus (3*R*)-Methylpyrrolidinol und (2*R*)-2-Cyclopentyl-2-hydroxy-2-phenylessigsäuremethylester. Zur Quarternierung wird Allylbromid verwendet.
$^1$H-NMR-Spektrum (300MHz, DMSO-$d_6$): δ (ppm) 7,62-7,56 (m, 2H, Phenyl). 7,39-7,31 (m, 2H, Phenyl), 7,29-7,22 (m, 1H), 6,14-5,91 (m, 1H), 5,82 (s, 1H, OH) 5,69-5,50 (m, 2H), 5,38 (m, 1H), 4,10 und 3,98 (d, 2H, $N^+$-$CH_2$-, zwei Diastereomere), 3,94-3,84 (m, 1H), 3,75-3,48 (m, 3H), 3,07 und 3,00 (s, 3H, $N^+$-Methyl, zwei Diastereomere), 2,95-2,83 (m, 1H, Cyclopentyl-Methin), 2,74-2,59 (m, 1H), 2,50 (DMSO) 2,19-2,01 (m, 1H), 1,68-1,13 (m, 8H, Cyclopentyl-Methylen) $^{13}$C-NMR (50 MHz, DMSO-$d_6$): δ (ppm) 173,3 (s, 1'-COO), 142,1 (s, Phenyl), 127,9 (d, Phenyl), 127,5 und 127,3 (t, zwei Diastereomere), 127,2 (d, Phenyl), 126,5 und 126,3 (d, zwei Diastereomere), 125,6 (d, Phenyl), 79,2 (s), 72,6 und 72,4 (d, zwei Diastereomere), 67,2 (t), 66,0 und 65,4 (t, $N^+$-$CH_2$-, zwei Diastereomere), 61,8 (t), 49,7 und 49,4 (q, $N^+$-Methyl, zwei Diastereomere), 46,6 (t, Cyclopentyl-Methin), 29,4 und 29,2 (t, zwei Diastereomere), 26,5/26,0/25,9/25,5 (4t, Cyclopentyl-Methylen),
Es liegt eine Mischung von zwei Diastereomeren vor, die sich in ihrer Konfiguration am quartären Stickstoff unterscheiden. Ihr Verhältnis beträgt 2,3:1, gemessen an den N-Methyl-Protonensignalen bei 3,07 und 3,0 ppm.

**Beispiel 11:**

(3*R*,2'*R*)-3-[(Cyclopentylhydroxyphenylacetyl)oxy]-1-methyl-1-propargyl-pyrrolidinium-bromid allg.Formel I (AR = Phenyl, $R_1$ = Cyclopentyl, $R_2$ = Propargyl, $R_3$ = Methyl, n = 1, A = Br); Die Herstellung erfolgt nach der unter Beispiel 5 angegebenen allgemeinen Vorschrift aus (3*R*)-Methylpyrrolidinol und (2*R*)-2-Cyclopentyl-2-hydroxy-2-phenylessigsäuremethylester. Zur Quarternierung wird Propargylbromid verwendet.
$^1$H-NMR-Spektrum (300MHz, DMSO-$d_6$): δ (ppm) 7,62-7,56 (m, 2H, Phenyl). 7,38-7,30 (m, 2H, Phenyl), 7,29-7,23 (m, 1H, Phenyl), 5,82 und 5,81 (s, 1H, OH, zwei Diastereomere) 5,41 (m, 1H), 4,50 und 4,42 (d, 2H, $N^+$-$CH_2$-, zwei Diastereomere), 4,09-4,05 (m, 1H), 4,03-3,93 (m, 1H), 3,84-3,56 (m, 3H), 3,32 ($H_2O$), 3,22 und 3,14 (s, 3H, $N^+$-Methyl, zwei Diastereomere), 2,98-2,83 (m, 1H, Cyclopentyl-Methin), 2,76-2,62 (m, 1H), 2,50 (DMSO) 2,21-2,06 (m, 1H), 1,64-1,12 (m, 8H, Cyclopentyl-Methylen)
$^{13}$C-NMR (50 MHz, DMSO-$d_6$): δ (ppm) 173,2 (s, 1'-COO), 142,0 (s, Phenyl), 127,9 (d, Phenyl), 127,2 (d, Phenyl), 125,6 (d, Phenyl), 82,4 (s), 79,2 (s), 72,7 und 72,5 (d, zwei Diastereomere), 67,5 (d), 67,3 (t), 62,2 (t), 53,7 und 53,4 (t, $N^+$-$CH_2$-, zwei Diastereomere), 50,2 (q, $N^+$-Methyl), 46,8 und 46,6 (t, Cyclopentyl-Methin, zwei Diastereomere), 29,7 und 29,4 (t, zwei Diastereomere), 26,6/ 26,5/25,9/25,5 (4t, Cyclopentyl-Methylen),
Es liegt eine Mischung von zwei Diastereomeren vor, die sich in ihrer Konfiguration am quartären Stickstoff unterscheiden. Ihr Verhältnis beträgt 2,3 : 1, gemessen an den N-Methyl- oder N-Methylen-Protonensignalen bei 3,22 und 3,14 bzw 4,55 und 4,47 ppm.

**Beispiel 12 (pharmakologische Daten):**

| Absolut-Konfiguration | AR | R1 | R2 | R3 | n | A | p$K_i$ $M_3$ | t ½ [min] $M_3$ |
|---|---|---|---|---|---|---|---|---|
| 3*R*,2'*R* | Phenyl | Cyclopentyl | Methyl | Methyl | 2 | Bromid | 9,64 | 69 |
| 3*R*,2'*S* | 2-Thienyl | Cyclopentyl | Methyl | Methyl | 1 | Bromid | 10,04 | 95 |
| 3*R*,2'*S* | 2-Thienyl | Cyclopentyl | Methyl | Methyl | 2 | Bromid | 9,76 | 90 |
| 3*R*,2'*R* | 4-Fluorphenyl | Cyclopentyl | Methyl | Methyl | 1 | Bromid | 9,64 | 57 |
| 3*R*,2'*S* | 4-Fluorphenyl | Cyclopentyl | Methyl | Methyl | 2 | Bromid | 9,51 | 73 |
| 3*R*,2'*R* | Phenyl | Cyclopentyl | Allyl | Methyl | 1 | Bromid | 9,69 | |
| 3*R*,2'*R* | Phenyl | Cyclopentyl | Propargyl | Methyl | 1 | Bromid | 9,78 | |
| 3*R*,2'*R* | Phenyl | Cyclopentyl | Trifluor-propyl | Methyl | 1 | Bromid | 9,54 | |

p$K_i$-Werte und Kinetik-Daten aus [$^3$H]-NMS-Bindungsstudien an humanen $M_3$-Rezeptorsubtypen aus CHO-K1-Zelllinien. Dissoziationshalbwertszeiten in Minuten.

**Patentansprüche**

1. Enantiomerenreine Ester der allgemeinen Formel I,

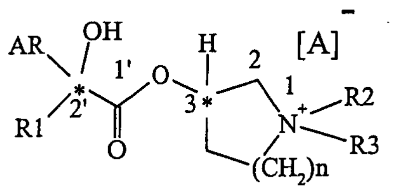

I

worin
R1 einen mono- oder bicyclischen $C_5$-$C_7$-Cycloalkylrest, der gegebenenfalls durch einen oder mehrere $C_1$-$C_3$-Alkyl-, $C_2$-$C_6$-Alkenyl- oder $C_2$-$C_6$-Alkinylrest(e) und/oder durch ein oder mehrere Halogenatom(e) wie Fluor, Chlor, Brom oder Iod substituiert ist;
R2 einen $C_1$-$C_6$-Alkyl-, $C_2$-$C_6$-Alkenyl- oder $C_2$-$C_6$-Alkinylrest, der gegebenenfalls durch ein oder mehrere Halogenatom(e) wie Fluor, Chlor, Brom, Iod substituiert sein kann;
R3 einen $C_1$-$C_3$-Alkylrest,
AR einen $C_6$-$C_{10}$-Aromaten oder einen Heteroaromaten, der Stickstoff, Schwefel oder Sauerstoff als Heteroatom enthält;
n eine ganze Zahl 1 oder 2;
$[A]^-$ ein Anion einer pharmakologisch unbedenklichen Säure
bedeuten können,
ausgenommen 3-[Cyclopentylhydroxyphenylacetyl)oxy]-1,1-dimethyl-pyrrolidinium (= Glycopyrronium),
und in welchen OH, AR und R1, bei Blickrichtung vom quartären Kohlenstoff-Zentrum zur Carboxylgruppe hin, im Uhrzeigersinn angeordnet sind.

2. Enantiomerenreine Ester der allgemeinen Formel I gemäß Anspruch 1, worin
R1 einen Cyclopentyl- (wenn n = 2 oder AR = Thienyl), einen Cyclohexyl- oder einen Norbornylrest;
R2 einen Methylrest;
R3 einen Methylrest;
AR einen Phenylrest oder Thienylrest;
n eine ganze Zahl 1 oder 2;
A Fluorid, Chlorid, Bromid oder Iodid
bedeuten können und OH, AR und R1 bei Blickrichtung gegen die Carboxylgruppe im Uhrzeigersinn angeordnet sind.

3. Ein pharmazeutisch geeignetes Salz von (3*R*,2'*R*)-3-[(Cyclohexylhydroxyphenylacetyl)oxy]-1,1-dimethyl-pyrrolidinium

4. Ein pharmazeutisch geeignetes Salz von (3*S*,2'*R*)-3-[(Cyclohexylhydroxyphenylacetyl)oxy]-1,1-dimethyl-pyrrolidinium

5. Ein pharmazeutisch geeignetes Salz von (3*R*,2'*S*)-3-[(Cyclopentylhydroxythienylacetyl)oxy]-1,1-dimethyl-pyrrolidinium

6. Ein pharmazeutisch geeignetes Salz von (3*S*,2'*S*)-3-[(Cyclopentylhydroxythienylacetyl)oxy]-1,1-dimethyl-pyrrolidinium

7. Ein pharmazeutisch geeignetes Salz von (3*R*,2'*S*)-3-[(Cyclopentylhydroxythienylacetyl)oxy]-1,1-dimethyl-piperidinium

8. Ein pharmazeutisch geeignetes Salz von (3*S*,2'*S*)-3-[(Cyclopentylhydroxythienylacetyl)oxy]-1,1-dimethyl-piperidinium

**9.** Verfahren zur Herstellung von Verbindungen der allgemeinen Formel I, ausgenommen 3-[Cyclopentylhydroxyphenylacetyl)oxy]-1,1-dimethyl-pyrrolidinium (= Glycopyrronium), gemäß Anspruch 1 oder 2, **dadurch gekennzeichnet, dass** man eine enantiomerenreine α-Hydroxycarbonsäure der allgemeinen Formel II in welcher OH, AR und R1, bei Blickrichtung vom quartären Kohlenstoff-Zentrum zur Carboxylgruppe hin, im Uhrzeigersinn angeordnet sind und

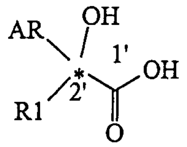

II

worin R1 und AR die in Anspruch 1 genannte Bedeutung haben, oder ihren Ester, bevorzugt einen $C_1$ - $C_3$ Alkylester, oder ein aktiviertes Säurederivat mit einem enantiomerenreinen Aminoalkohol der allgemeinen Formel III (*R*- oder *S*-Enantiomer)

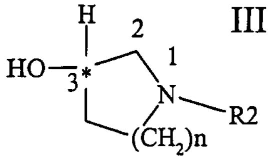

III

worin R2 und n die in Anspruch 1 genannte Bedeutung haben, umsetzt und die so erhaltenen enantiomerenreinen Ester der allgemeinen Formel IV

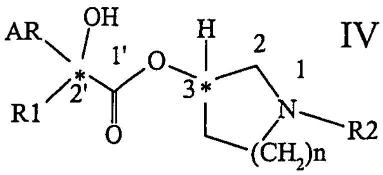

IV

mit einem Alkylierungsmittel der allgemeinen Formel

R3 - X,

in der X eine gegen eine tertiäre Aminogruppe substituierbare Austrittsgruppe verkörpert, umsetzt und das resultierende Salz isoliert bzw. umsalzt.

**10.** Verfahren zur Herstellung von Verbindungen der allgemeinen Formel I, ausgenommen 3-[Cyclopentylhydroxyphenylacetyl)oxy]-1,1-dimethyl-pyrrolidinium (= Glycopyrronium), gemäß Anspruch 1 oder 2, **dadurch gekennzeichnet, dass** man eine racemische α-Hydroxycarbonsäure der allgemeinen Formel II

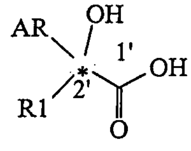

II

worin R1 und AR die in Anspruch 1 genannte Bedeutung haben, oder ihren Ester oder ein aktiviertes Säurederivat mit einem enantiomerenreinen Aminoalkohol der allgemeinen Formel III (R-oder S-Enantiomer)

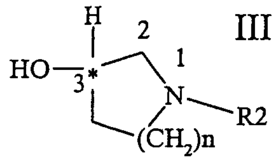

III

worin R2 und n die in Anspruch 1 genannte Bedeutung haben, umsetzt, das resultierende Diastereomerengemisch nach an sich aus dem Stand der Technik bekannten Verfahren - insbesondere auf dem Wege der Kristallisation, bevorzugt unter Verwendung einer enantiomerenreinen Hilfssäure - trennt, die so erhaltenen Ester der allgemeinen Formel IV, in welchen OH, AR und R1, bei Blickrichtung vom quartären Kohlenstoff-Zentrum zur Carboxylgruppe hin, im Uhrzeigersinn angeordnet sind

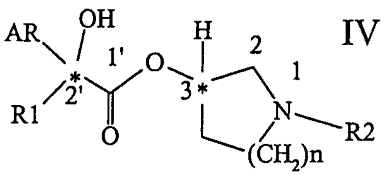

IV

mit einem Alkylierungsmittel der allgemeinen Formel

R3 - X,

in der X eine gegen eine tertiäre Aminogruppe substituierbare Austrittsgruppe verkörpert, umsetzt und das resultierende Salz isoliert bzw. umsalzt.

**11.** Arzneimittel enthaltend eine Verbindung nach einem der Ansprüche 1 - 8, nebst pharmazeutischen Hilfs- und/oder Trägerstoffen

**12.** Verwendung von Verbindungen der allgemeinen Formel I gemäß Ansprüchen 1 - 8 zur Herstellung eines Arzneimittels zur Behandlung von Spasmen der glatten Muskulatur des Magen-Darm-Kanals oder des Urogenitaltraktes, sowie zur Behandlung obstruktiver Atemwegserkrankungen, bevorzugt Asthma bronchiale oder chronische Bronchitis.

**13.** Verwendung eines pharmazeutisch geeigneten Salzes von (3*R*,2'*S*)-3-[(Cyclopentylhydroxythienylacetyl)oxy]-1,1-dimethyl-pyrrolidinium oder von (3*R*,2'*S*)-3-[(Cyclopentylhydroxythienylacetyl)-oxy]-1,1-dimethyl-piperidinium nach Anspruch 12 zur Herstellung eines Arzneimittels zur Behandlung von Spasmen der glatten Muskulatur des Magen-Darm-Kanals oder des Urogenitaltraktes, sowie zur Behandlung obstruktiver Atemwegserkrankungen.

**14.** Verwendung eines pharmazeutisch geeigneten Salzes von (3*R*,2'*S*)-3-[(Cyclopentylhydroxythienylacetyl)oxy]-1,1-dimethyl-pyrrolidinium oder von (3*R*,2'*S*)-3-[(Cyclopentylhydroxythienylacetyl)-oxy]-1,1-dimethyl-piperidinium nach Anspruch 13 zur Herstellung eines Arzneimittels zur Behandlung von Asthma bronchiale oder chronischer Bronchitis

**15.** Verwendung nach Anspruch 12 von Enantiomeren der allgemeinen Formel I gemäß einem der Ansprüche 1 - 8 mit hoher $M_3$-Subtypaffinität und langen Dissoziationshalbwertszeiten am $M_3$-Rezeptorsubtyp zur Herstellung von Arzneimitteln zur Behandlung von Spasmen der glatten Muskulatur des Magen-Darm-Kanals oder des Urogenitaltraktes, sowie zur Behandlung obstruktiver Atemwegserkrankungen, bevorzugt Asthma bronchiale oder chronische Bronchitis.

**16.** Verwendung eines pharmazeutisch geeigneten Salzes von (3*R*,2'*S*)-3-[(Cyclopentylhydroxythienylacetyl)oxy]-1,1-dimethyl-pyrrolidinium oder von (3*R*,2'*S*)-3-[(Cyclopentylhydroxythienylacetyl)-oxy]-1,1-dimethyl-piperidinium nach Anspruch 15 zur Herstellung eines Arzneimittels mit hoher $M_3$-Subtypaffinität und langen Dissoziationshalbwertszeiten am $M_3$-Rezeptorsubtyp zur Behandlung von Spasmen der glatten Muskulatur des Magen-Darm-Kanals oder des Urogenitaltraktes sowie zur Behandlung obstruktiver Atemwegserkrankungen.

**17.** Verwendung eines pharmazeutisch geeigneten Salzes von (3*R*,2'*S*)-3-[(Cyclopentylhydroxythienylacetyl)oxy]-1,1-dimethyl-pyrrolidinium oder von (3*R*,2'*S*)-3-[(Cyclopentylhydroxythienylacetyl)-oxy]-1,1-dimethyl-piperidinium nach Anspruch 16 zur Herstellung eines Arzneimittels zur Behandlung von Asthma bronchiale oder chronischer Bronchitis

Europäisches Patentamt

# EUROPÄISCHER RECHERCHENBERICHT

Nummer der Anmeldung
EP 03 00 5233

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| Y | "CHEMICAL ABSTRACTS + INDEXES, AMERICAN CHEMICAL SOCIETY. COLUMBUS, US" CHEMICAL ABSTRACTS + INDEXES, AMERICAN CHEMICAL SOCIETY. COLUMBUS, US, Bd. 11, Nr. 119, 1993, Seite 82 XP002055749 ISSN: 0009-2258 & FUDER ET AL: "Glycopyrronium bromide blocks differentially responses mediated by muscarinic receptor subtypes" NAUNYN-SCHMIEDEBERG'S ARCH. PHARMACOL., Bd. 347, Nr. 6, 1993, Seiten 591-595, --- | 1-17 | C07D207/12 C07D211/40 C07D409/12 A61K31/40 A61K31/4025 A61K31/452 A61K31/4535 A61P11/00 A61P21/00 |
| Y | WO 96 33973 A (BANYU PHARMA CO LTD ;KAWAKAMI KUMIKO (JP); NOMOTO TAKASHI (JP); OH) 31. Oktober 1996 (1996-10-31) * Zusammenfassung * | 1-17 | |
| E | & EP 0 823 423 A (BANYU PHARMA CO LTD) 11. Februar 1998 (1998-02-11) * Seite 1, Zeile 46 - Zeile 51; Ansprüche; Beispiele 22,25,28,29,35,55,70,71; Tabellen 1-3 * ----- | | RECHERCHIERTE SACHGEBIETE (Int.Cl.7) C07D A61K |

Der vorliegende Recherchenbericht wurde für alle Patentansprüche erstellt

| Recherchenort | Abschlußdatum der Recherche | Prüfer |
|---|---|---|
| MÜNCHEN | 19. September 2003 | Hanisch, I |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 03 00 5233

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-09-2003

| Im Recherchenbericht angeführtes Patentdokument | Datum der Veröffentlichung | Mitglied(er) der Patentfamilie | Datum der Veröffentlichung |
|---|---|---|---|
| WO 9633973 A | 31-10-1996 | AU 700837 B2 | 14-01-1999 |
| | | AU 5513996 A | 18-11-1996 |
| | | CA 2218479 A1 | 31-10-1996 |
| | | EP 0823423 A1 | 11-02-1998 |
| | | WO 9633973 A1 | 31-10-1996 |
| | | JP 2993124 B2 | 20-12-1999 |
| | | US 5750540 A | 12-05-1998 |

EPO FORM P0461

Für nähere Einzelheiten zu diesem Anhang : siehe Amtsblatt des Europäischen Patentamts, Nr.12/82